# EUROPEAN PATENT APPLICATION

(11) **EP 2 133 351 A2**
(43) Date of publication of application: **16.12.2009**
(21) Application number: 09170610.1
(22) Date of filing: 14.08.2007
(51) Int. Cl.: C07D 471/04

(54) **Crystal form of 9-hydroxy-risperidone (paliperidone)**

(30) Priority: 14.08.2006 US 837804 P; 10.05.2007 US 928745 P; 10.05.2007 US 928747 P; 15.05.2007 US 930392 P; 14.06.2007 US 929126 P; 05.07.2007 US 958571 P; 10.07.2007 US 929703 P; 26.07.2007 US 935094 P; 26.07.2007 US 935093 P; 07.08.2007 US 963922 P
(62) Divisional of application: 07811307.3
(71) Applicant: TEVA PHARMACEUTICAL INDUSTRIES LTD., 49131 Petah Tiqva (IL)
(72) Inventor: Santiago, Ini, 34404, Haifa (IL); Chen, Kobi, Kfar-saba (IL); Lancry, Eli, Ashdod (IL); Chasid, Naama, 49453, Petah Tikva (IL); Shmuely, Yaron, 38303, Hedera (IL); Doiltzky, Ben-Zion, Petach Tiqva (IL)
(74) Representative: Russell, Tim

(57) **Abstract**

The present invention provides a crystalline form of Paliperidone, and processes for preparing thereof.

## Description

### FIELD OF INVENTION

The present invention is related to crystalline forms of 9-hydroxy-risperidone (paliperidone) and methods of preparation thereof.

### BACKGROUND OF THE INVENTION

RISPERDAL® (risperidone) is a psychotropic agent belonging to the chemical class of benzisoxazole derivatives. The chemical designation is 3-[2-[4-(6-fluoro-1,2-benzisoxazol-3-yl)-1-piperidinyl]ethyl]-6,7,8,9-tetrahydro-2-methyl-4H-pyrido[1,2-a]pyrimidin-4-one.

Risperidone is a selective monoaminergic antagonist which has affinity for serotonin-5-HT2, dopamine-D2, H1-histamine, alpha1- and alpha2-adrenergic receptors. Risperidone has no affinity for cholinergic receptors. It is a potent D2-antagonist. This active pharmaceutical ingredient is metabolized by cytochrome P-450 IID6 to produce 9-hydroxy-risperidone, also known as Paliperidone, which has a similar pharmacological activity to risperidone.

Paliperidone, 3-[2-[4-(6-fluorobenzo[d]isoxazol-3-yl)-1-piperidyl]ethyl]-7-hydroxy-4-methyl-1,5-diazabicyclo[4.4.0]deca-3,5-dien-2-one, is a 5-HT antagonist belonging to the chemical class of benzisoxazole derivatives and a racemic mixture having the following structural formula:

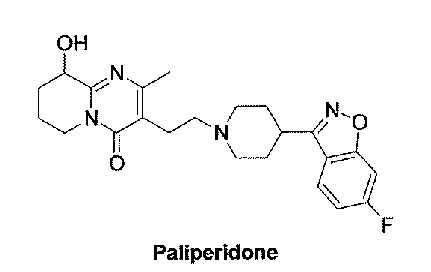

Paliperidone is a metabolite of Risperidone. Marketed under the name, Invega^{®}, Paliperidone is a psychotropic agent approved in the United States for the treatment of schizophrenia.

Paliperidone is described in U.S. Patent No. 5,158,952. U.S. Patent No. 5,254,556 describes a process for Paliperidone synthesis.

Polymorphism, the occurrence of different crystal forms, is a property of some molecules and molecular complexes. A single molecule, like 9-hydroxy-risperidone, may give rise to a variety of crystalline forms having distinct crystal structures and physical properties.

The difference in the physical properties of different crystalline forms results from the orientation and intermolecular interactions of adjacent molecules or complexes in the bulk solid. Accordingly, polymorphs are distinct solids sharing the same molecular formula yet having distinct advantageous physical properties compared to other crystalline forms of the same compound or complex.

The discovery of new polymorphic forms of a pharmaceutically useful compound provides a new opportunity to improve the performance characteristics of a pharmaceutical product. It enlarges the repertoire of materials that a formulation scientist has available for designing, for example, a pharmaceutical dosage form of a drug with a targeted release profile or other desired characteristic.

There is a need in the art for polymorphic forms of 9-hydroxy-risperidone, Paliperidone.

### SUMMARY OF THE INVENTION

An embodiment of the present invention provides crystalline Paliperidone Form V, characterized by data selected from the group consisting of
(i) X-ray powder diffraction spectrum with four or more peaks from the list of: about 9.8, 10.9, 15.8, 21.2 and 21.6 degrees two theta ± 0.2 degrees two theta;
(ii) a solid-state ¹³C NMR spectrum with signals at about 163.4, 161.4 and 157.9 ± 0.2 ppm; and
(iii)a solid-state ¹³C NMR spectrum having chemical shifts differences between the signal exhibiting the lowest chemical shift and another in the chemical shift range of 100 to 180 ppm of about 51.1, 49.1 and 45.6 ± 0.1 ppm.

The present invention also provides pure or substantially pure crystalline Form V of Paliperidone.

The present invention also provides processes for preparing crystalline Form V of Paliperidone.

### BRIEF DESCRIPTION OF THE DRAWINGS

Figure 1 illustrates the PXRD pattern for substantially pure crystalline 9-hydroxy-risperidone Form I of the present invention, wherein the unit for the vertical axis is cps and the unit for the horizontal axis is degree two theta.

Figure 2 illustrates the PXRD pattern for crystalline 9-hydroxy-risperidone Form II of the present invention, wherein the unit for the vertical axis is cps and the unit for the horizontal axis is degree two theta.

Figure 3 illustrates the PXRD pattern for crystalline 9-hydroxy-risperidone Form III of the present invention, wherein the unit for the vertical axis is cps and the unit for the horizontal axis is degree two theta.

Figure 4 illustrates the PXRD pattern for 9-hydroxy-risperidone Form IV of the present invention, wherein the unit for the vertical axis is cps and the unit for the horizontal axis is degree two theta.

Figure 5 illustrates the PXRD pattern for crystalline 9-hydroxy-risperidone Form V of the present invention, wherein the unit for the vertical axis is cps and the unit for the horizontal axis is degree two theta.

Figures 6 and 7 illustrate solid-state ¹³C NMR spectrum of Paliperidone Form V in the 110-180 ppm range and in the 0-180 range.

Figure 8 illustrates the PXRD pattern for pure crystalline 9-hydroxy-risperidone Form VI of the present invention, wherein the unit for the vertical axis is cps and the unit for the horizontal axis is degree two theta.

Figure 9 illustrates the PXRD pattern for crystalline 9-hydroxy-risperidone Form VI of the present invention, wherein the unit for the vertical axis is cps and the unit for the horizontal axis is degree two theta.

Figure 10 is a table of PXRD peaks for Paliperidone crystalline forms which may be used in determining the percent contamination of a certain Paliperidone crystalline form by other Paliperidone crystalline forms.

### DETAILED DESCRIPTION OF THE INVENTION

As used herein, 9-hydroxy-risperidone is interchangeable with Paliperidone.

As used herein, "room temperature" means a temperature of about 18°C to about 26°C. Preferably, "room temperature" means about 20°C to about 25°C.

As used in this patent application, "overnight" preferably means a duration of about 12 hours to about 18 hours.

As used herein, the percent of crystalline form in amorphous material is quantified by methods known in the art using a "crystallinity index" available in most XRD software. This index is used to measure the percent of crystalline contamination within the amorphous material.

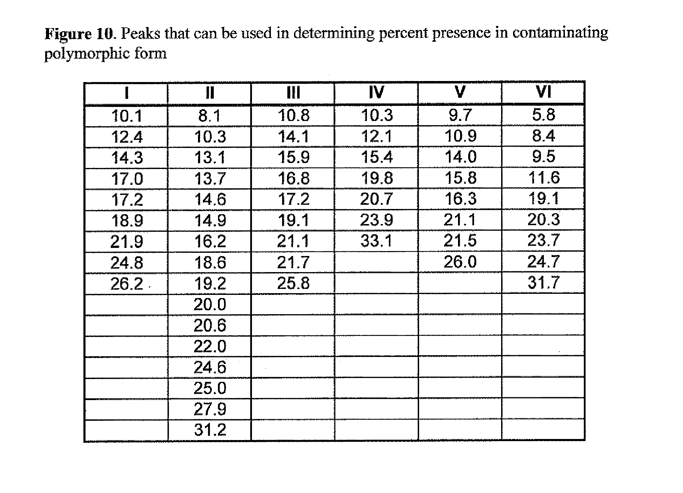

With regard to determining percent of one or more crystalline forms present in another, a person skilled in the art can select a characterizing peak or a number of peaks from the known crystalline form (see Figure 10 for reference) and quantify it following the known art. The XRD analysis for quantitation purposes can be performed according, for instance, to the European Pharmacopoeia version 5.6, "Characterization of crystalline solids by XRPD", pg 4432-4437.

As used herein, the term chemical shift difference refers to the difference in chemical shifts between a reference signal and another signal in the same NMR spectrum. In the present patent application the chemical shift differences were calculated by subtracting the chemical shift value of the signal exhibiting the lowest chemical shift (reference signal) in the solid state ¹³CNMR spectrum in a specific range (for example 100 to 180 ppm) from chemical shifts values of another (observed) signal in the same NMR spectrum in the range. These chemical shift differences are to provide a measurement for a substance, compensating for a phenomenon in NMR spectroscopy wherein, depending on the instrumentation, temperature, and calibration method used, a shift in the solid-state NMR "fingerprint" is observed. This shift in the solid-state NMR "fingerprint", having chemical shift resonances at a certain positions, is such that although the individual chemical shifts of signals have altered, the difference between chemical shifts of each signal and another is retained.

In another embodiment, the present invention provides amorphous Paliperidone.

The present invention further provides a process for the preparation of substantially pure amorphous paliperidone comprising: providing a solution of paliperidone and dichloromethane, and removal of the solvent to obtain amorphous paliperidone. The solution of paliperidone and dichloromethane may be prepared by combining Paliperidone and dichloromethane and heating for a period to allow complete dissolution. The solvent may be removed through evaporation for example by maintaining at a temperature from about 35°C, preferably under reduced pressure or alternatively by spray drying. When using the spray drying technique, the paliperidone solution can be sprayed in a chamber with ambient nitrogen at a co-current flow. The spray rate of the solution is preferably at about 5.6 ml/min. Further, the co-current flow of nitrogen may vary between about 70°C and about 120°C at 34 m³/h. To obtain the amorphous paliperidone in such process, the temperature for the outlet solids from such chamber may be from about 45°C to about 80°C.

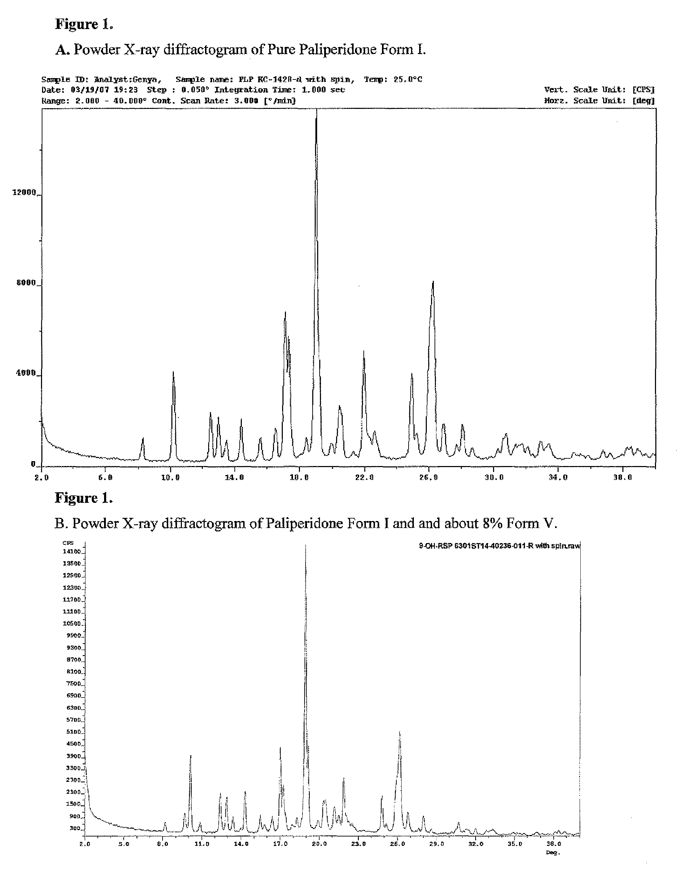

In another embodiment, the present invention provides crystalline 9-hydroxy-risperidone (paliperidone), characterized by X-ray powder diffraction reflections at about: 10.1, 12.4, 14.3, 17.0 and 17.2 ± 0.2 degrees two theta, designated as a Form I. The crystalline paliperidone can be further characterized by one or more X-ray powder diffraction peaks at about 12.9, 18.9, 21.9, 24.8 and 26.2 ± 0.2 degrees two-theta. A typical powder x-ray diffractogram pattern for a mixture of crystalline paliperidone containing Form I is shown in Figure 1B. The crystalline form may have a melting point of about 166 to 167°C.

The crystalline Form I can have solid-state ¹³C NMR spectrum with signals at about 163.1, 161.2 and 156.8 ± 0.2 ppm or a solid-state ¹³C NMR spectrum having chemical shifts differences between the signal exhibiting the lowest chemical shift and another in the chemical shift range of 115 to 180 ppm of about 45.8, 43.9 and 39.5 ± 0.1 ppm. In addition, the solid-state ¹³C NMR spectrum of Form I may have one or more signals at about 121.2 and 117.3 ± 0.2 ppm. In addition, the solid-state ¹³C NMR spectrum of Form I may have chemical shift differences between the signal exhibiting the lowest chemical shift and another in the chemical shift range of 115 to 180 ppm of about 45.8, 43.9, 39.5 and 3.9 ± 0.1 ppm. The signal exhibiting the lowest chemical shift in the chemical shift range of 115 to 180 ppm is, typically, at about 117.3 ± 1ppm. Form I is preferably anhydrous, showing weight loss of about 0.6% (between 25-168°C), as measured by TGA. The water content of Form I is about 0.5%, as measured by KF titration.

Form I may be prepared by recrystallization from acetonitrile or methanol. The present invention provides a process for preparing Form I, comprising dissolving Paliperidone in at least one solvent selected from acetonitrile and methanol to form a solution; and precipitation of Paliperidone from the solution to obtain Paliperidone Form I. Preferably, the dissolving step is performed by heating Paliperidone in the at least one solvent to reflux. Preferably, the precipitation is conducted by removing the solvent via evaporation (such as under reduced pressure) or cooling.

The present invention also provides a process for preparing Form I, comprising slurrying solid Paliperidone in at least one solvents selected from the group consisting of ethanol/water (volume ratio about 1:1), acetone/water (volume ratio of about 1:1), dichlorobenzene, isopropanol, water and acetone for a duration of at least about 30 minutes, preferably ranging from about 12 hours to about 48 hours, at a temperature of about room temperature to about 60°C to obtain Form I. As used in this patent application, "about 1:1" refers to a ratio X:Y, wherein X ranges from 0.8 to 1.2 and Y ranges from 0.8 to 1.2.

The present invention also provides a process for preparing Form I, comprising dissolving Paliperidone in dichloromethane by heating to form a heated solution via complete dissolution; adding the heated solution dropwise into hexane maintained at a temperature of room temperature or less, e.g., cooled at a temperature of about 0°C to about 5°C, to precipitate Paliperidone as Form I. Preferably, one volume of the heated solution is added dropwise into about 3 volumes of the cooled hexane. The hexane is cooled preferably in an ice bath.

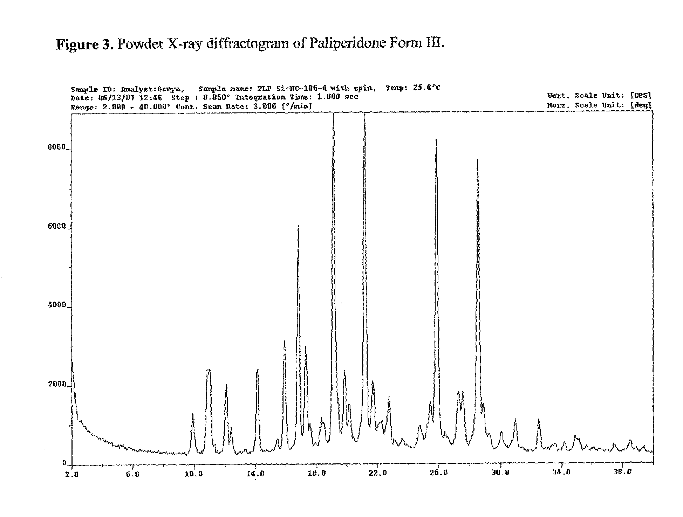

In another embodiment, paliperidone Form I is prepared as a mixture of Form I and V, for instance, a mixture of Form I having about 8% by weight of paliperidone Form V with the PXRD pattern as substantially shown in Figure 3B. In another embodiment, the present invention presents substantially pure crystalline 9-hydroxy-risperidone Form I. The substantially pure crystalline 9-hydroxy-risperidone Form I contains less than 8% and preferably less than 5% of other crystalline forms of paliperidone. An example of the PXRD pattern for the substantially pure Form I is as substantially depicted in Figure 3A.

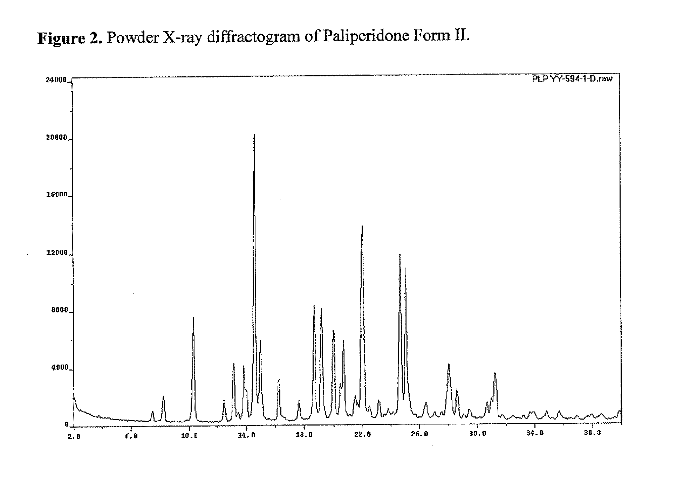

In one embodiment, the present invention provides a crystalline form of 9-hydroxy-risperidone (paliperidone), characterized by X-ray powder diffraction (PXRD) reflections at about: 10.3, 14.6, 22.0, 24.6 and 25.0 degrees two theta ± 0.2 degrees two theta or alternatively characterized by X-ray powder diffraction (PXRD) reflections at about: 10.3, 13.3, 13.9, 14.6 and 15.1 degrees two theta ± 0.2 degrees two theta designated as Form II. This form can be further characterized by any one or more of the X-ray powder diffraction reflections selected from the list of: 13.1, 13.8, 14.1, 18.7 and 28.0 degrees two-theta, ± 0.2 degrees two-theta. An example of the powder x-ray diffraction pattern for the crystalline Form II is shown in Figure 2. Preferably, the above Form II is preferably anhydrous, showing weight loss of about 0.4% (between 25-145 °C), as measured by TGA. The water content of this Form II is about 0.5%, as measured by KF titration.

The crystalline Form II can alternatively be characterized by a solid-state ¹³C NMR spectrum with signals at about 163.4, 121.8 and 116.7 ± 0.2 ppm ± 0.2 ppm or a solid-state ¹³C NMR spectrum having chemical shifts differences in the 95 to 180 ppm range between the signal exhibiting the lowest chemical shift in this range and another in the chemical shift range of about 65.7, 24.1 and 19.0 ± 0.1 ppm. In addition, the solid-state ¹³C NMR spectrum of Form II may have one or more signals at about 163.4, 156.2, 121.8, 116.7 and 97.7 ± 0.2 ppm. In addition, the solid-state ¹³C NMR spectrum of Form II may have chemical shift differences between the signal exhibiting the lowest chemical shift and another in the chemical shift range of 95 to 180 ppm of about 65.7, 58.5, 24.1 and 19.0 ± 0.1 ppm ± 0.1 ppm. The signal exhibiting the lowest chemical shift in the chemical shift range between 95 to 180 ppm is typically, at about 97.7 ± 1ppm.

The present invention further provides a process for the preparation of crystalline Form II of paliperidone comprising crystallization from a solution of paliperidone and a solvent selected from a group consisting of: ethanol, methanol, n-propanol, isopropanol, n-butanol, 2-butanol, isobutanol, 2-pentanol, n-pentanol, ethanol/water (volume ratio ranging from about 1:1 to about 3:1), isopropanol/water (volume ratio ranging from about 1:1 to about 3:1), acetonitrile, toluene, chlorobenzene, dichlorobenzene, 1,2-dichloroethane, isobutyl acetate, n-butyl acetate, ethyl acetate/water (volumn ratio ranging from about 1:1 to about 3:1), diethyl carbonate, acetone, acetone/water (volume ratio ranging from about 1:1 to about 3:1), methyl ethyl ketone (MEK), methyl isopropyl ketone (MIPK), methyl isobutyl ketone (MIBK), dibutyl ether, polyglycol methyl ether (PGME), dioxane, propylene glycol, Cellosolve, tetrahydrofuran (THF), dimethyl formamide (DFM), dimethyl acetamide (DMA), dimethyl sulfoxide (DMSO) and DMC.

The present invention further provides a process for the preparation of crystalline Form II of paliperidone, comprising providing a solution of Paliperidone in dichloromethane; adding at least one anti-solvent selected from the group consisting of methyl t-butyl ether (MTBE), methyl ethyl ketone (MEK), ethyl acetate, acetonitrile, cyclohexane, heptane, toluene and butanol to precipitate Paliperidone as Form II. The solution of Paliperidone is preferably prepared by heating Paliperidone in dichloromethane to reflux. In an embodiment of the process, the Paliperidone solution is cooled before the addition of the at least one anti-solvent. Preferably, the at least one anti-solvent is added gradually, e.g., dropwise. Alternatively, the at least one anti-solvent is added at once, especially when the at least one anti-solvent is cooled to below room temperature before addition to the dichloromethane solution of Paliperidone. Preferably, during and/or after the addition of the at least one anti-solvent, the mixture is stirred, more preferably, at about room temperature. The stirring is preferably for about I hour to 3 hours, more preferably about 1.5 hour.

In the above processes for preparing Form II, the ingredients may be heated in order to achieve dissolution. Stirring may also be employed to promote dissolution. Preferably, the ingredients are heated to reflux. Whether the ingredients are heated, the process may further comprise cooling, to induce crystallization.

Crystallization is often by cooling. Cooling may be to a temperature of about -10°C to about 25°C. The paliperidone crystalline form may be recovered by any method known to the skilled artisan. Preferably, the paliperidone crystalline form is recovered from the mixture by filtration, and then dried under reduced pressure (< 1 atmosphere).

In another embodiment, the present invention provides a crystalline form of 9-hydroxy-risperidone, characterized by X-ray powder diffraction reflections at about: 10.8, 14.1, 15.8 and 16.8 degrees two theta ± 0.2 degrees two theta, designated as Form III. In another embodiment, the crystalline Form III is further characterized by X-ray powder diffraction reflections at about 25.8. In yet another embodiment, this crystalline form is further characterized by one or more X-ray powder diffraction reflections selected from the list of about 9.9, 11.0, 12.0, 17.3 and 32.5 degrees two theta ± 0.2 degrees two theta. A typical powder x-ray diffraction diagram for the crystalline Form III is shown in Figure 3.

The crystalline Form III can alternatively be characterized by a solid-state ¹³C NMR spectrum with signals at about 164.1, 161.3 and 157.9 ± 0.2 ppm or a solid-state ¹³C NMR spectrum having chemical shifts differences between the signal exhibiting the lowest chemical shift within the range and another in the chemical shift range of 115 to 180 ppm of about 46.7, 43.9 and 40.5 ± 0.1 ppm. In addition, the solid-state ¹³C NMR spectrum may have one or more signals at about 164.1, 161.3, 157.9, 123.9 and 117.4 ± 0.2 ppm. In addition, the solid-state ¹³C NMR spectrum of Form III may have chemical shift differences between the signal exhibiting the lowest chemical shift and another in the chemical shift range of 115 to 180 ppm of about 46.7, 443.9, 40.5 and 6.5 ± 0.1 ppm. The signal exhibiting the lowest chemical shift in the chemical shift range between 115 to 180 ppm is typically at about 117.4 ± 1 ppm.

Preferably, the above Form III is an NMP solvate, showing weight loss of about 19.2% (between 25-168°C), as measured by TGA. Preferably, the Form III is a monosolvate of NMP. The water content of this Form III is about 0.2%, as measured by KF titration.

The present invention further provides a process for the preparation of crystalline Form III of Paliperidone comprising providing a solution of paliperidone and 1-methyl-2-pyrrolidone (NMP) and crystallizing for example by cooling to obtain the Paliperidone Form III.

In the process for preparing Form III, the ingredients may be heated in order to achieve dissolution. Stirring may also be employed to promote dissolution. Preferably, the ingredients are heated to reflux. Whether the ingredients are heated, the process may further comprise cooling, to induce crystallization.

The present invention further provides a process for the preparation of crystalline Form III of Paliperidone, comprising stirring Paliperidone Form II solid in about 10 volumes of N-methyl 2-pyrrolidone (NMP) at about 50°C to about 65°C for about 12 hours to about 30 hours, preferably about 24 hours to convert Form II to Form III.

The paliperidone crystalline Form III may be recovered by any method known to the skilled artisan. Preferably, the paliperidone crystalline form is recovered from the mixture by filtration, and then dried under reduced pressure (< 1 atmosphere).

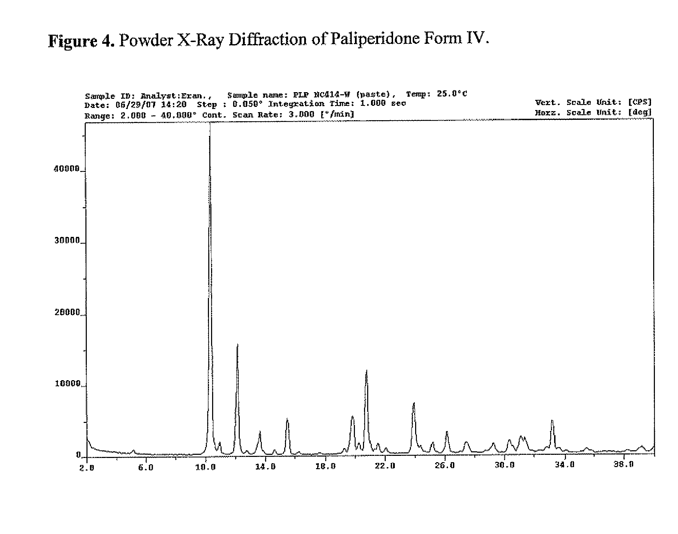

In another embodiment, the present invention provides a crystalline form of 9-hydroxy-risperidone, characterized by X-ray powder diffraction reflections at about: 10.2, 12.2 and 15.5 degrees two theta ± 0.2 degrees two theta, designated as Form IV. Form IV can be further characterized by an additional X-ray powder diffraction reflections at about 13.6 degrees two theta ± 0.2 degrees two theta. Optionally, this crystalline form can be further characterized by X-ray powder diffraction reflections at about 23.9 and 33.2 degrees two theta, 0.2 degrees two theta. A typical powder x-ray diffraction diagram for the crystalline Form IV is shown in Figure 4.

The crystalline Form IV can alternatively be characterized by a solid-state ¹³C NMR spectrum with signals at about 162.6, 160.5 and 157.6 ± 0.2 ppm or a solid-state ¹³C NMR spectrum having chemical shifts differences between the signal exhibiting the lowest chemical shift and another in the chemical shift range of 115 to 180 ppm of about 45.9, 43.8 and 40.9 ± 0.1 ppm. In addition, the solid-state ¹³C NMR spectrum may have one or more signals at about 162.6, 160.5, 157.6, 118.6 and 116.7 ± 0.2 ppm. In addition, the solid-state ¹³C NMR spectrum for Form IV may have chemical shift differences between the signal exhibiting the lowest chemical shift and another in the chemical shift range of 115 to 180 ppm of about 45.9, 43.8, 40.9 and 1.9 ± 0.1 ppm ± 0.1 ppm. The signal exhibiting the lowest chemical shift in the chemical shift range between 115 to 180ppm is typically at about 116.7 ± 1ppm.

The present invention further provides a process for the preparation of crystalline Form IV of paliperidone comprising crystallization from a solution of paliperidone and a solvent selected from the group consisting of dioxane, a mixture of acetone/water (volume ratio ranging from about 3:1 to about 5:1), and methanol/water (volume ratio ranging from about 3:1 to about 5:1).

In the processes for preparing Form IV, the ingredients may be heated in order to achieve dissolution. Stirring may also be employed to promote dissolution. Preferably, the ingredients are heated to reflux. Where the ingredients are heated, the process may further comprise cooling, to induce crystallization. The crystalline Form IV of paliperidone is recovered from the mixture by filtration.

The present invention further provides a process for the preparation of crystalline Form IV of Paliperidone, comprising providing a solution of Paliperidone in n-propanol or dioxane; and adding water as an anti-solvent to the solution to induce precipitation of Paliperidone as Form IV.

The present invention further provides a process for the preparation of crystalline Form IV of Paliperidone, comprising providing a solution of Paliperidone in about 30 to about 50 volumes, i.e., about one gram/30 ml to about one gram/50 ml), preferably about 40 volumes, of acetone/water (volume ratio ranging from about 1;1 to about 3:1) by heating to reflux; after Paliperidone is completely dissolved the hot solution is filtrated through hi-flow; and the temperature of the solution is reduced, e.g., by cooling the solution, preferably to a temperature of about 0°C to about 5°C, induce precipitation of Form IV.

In yet another embodiment, a process is presented for preparing Form IV comprising exposing Paliperidone Form III to moist environment such as at least 60% to 80% relative humidity for a sufficient period to allow for conversion. The period of time necessary can be determined by periodic analyses of PXRDs.

In another embodiment, the present invention provides a crystalline form of 9-hydroxy-risperidone, characterized by 4 or more X-ray powder diffraction reflections from the following list, about: 9.8, 10.9, 15.8, 21.2 and 21.6 degrees two theta ± 0.2 degrees two theta, designated as Form V. Form V can be further characterized by additional X-ray powder diffraction reflections at about: 14.1, 18.0 and/or 26.0 degrees two theta ± 0.2 degrees two theta.

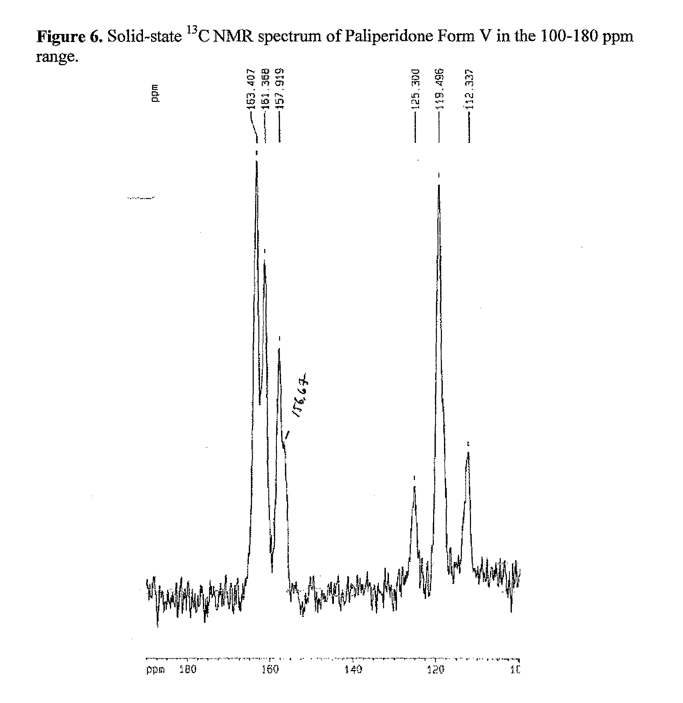

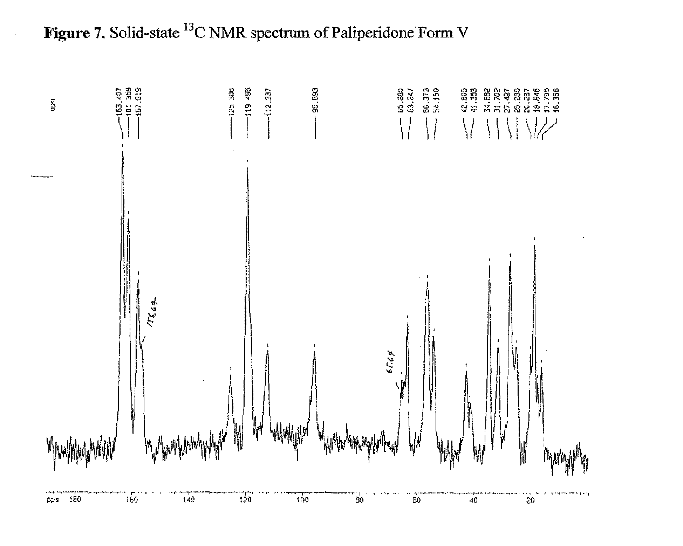

The crystalline Form V can alternatively be characterized by a solid-state ¹³C NMR spectrum with signals at about 163.4, 161.4 and 157.9 ± 0.2 ppm or a solid-state ¹³C NMR spectrum having chemical shifts differences between the signal exhibiting the lowest chemical shift and another in the chemical shift range of 100 to 180 ppm of about 51.1, 49.1 and 45.6 ± D.1 ppm. In addition, the solid-state ¹³C NMR spectrum of Form V may have one or more signals at about 163.4, 161.4, 157.9, 119.5 and 112.3 ± 0.2 ppm. In addition, the solid-state ¹³C NMR spectrum of Form V may have chemical shift differences between the signal exhibiting the lowest chemical shift and another in the chemical shift range of 100 to 180 ppm of about 51.1, 49.1, 45.6 and 7.2 ± 0.1 ppm. A typical ¹³C NMR spectrum for Form V is depicted in Figures 6 and 7. The signal exhibiting the lowest chemical shift in the chemical shift range between 100 to 180 ppm is typically at about 112.3 ± 1ppm.

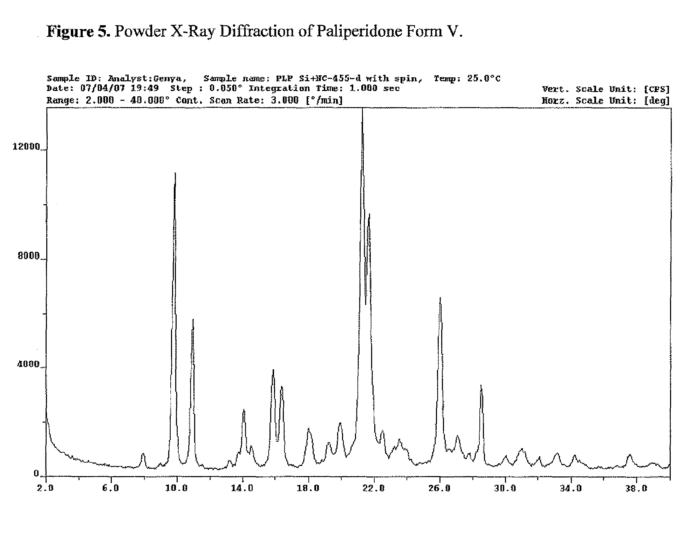

A typical powder x-ray diffraction diagram for the crystalline Form V is shown in Figure 5. Preferably, the above Form V is anhydrous, showing weight loss of about 0.1% (between 25-155°C), as measured by TGA. The water content of this Form V is about 0.3%, as measured by KF titration.

Form V may be prepared by crystallization from acetonitrile.

In one embodiment of the present invention, the crystalline paliperidone Form V is pure. The Form V has high crystalline purity, wherein the crystalline paliperidone form V contains less than 20%, preferably less than 10%, more preferably less than 5%, and most preferably less than 1%, of any one of the other crystalline forms of paliperidone. Specifically, the Form V has high crystalline purity, wherein the crystalline paliperidone Form V contains less than 20%, preferably less than 10%, more preferably less than 5%, and most preferably less than 1%, of any one of Paliperidone Forms I and II. The pure Form V can have less than 20%, preferably less than 10%, more preferably less than 5%, and most preferably less than 1%, of other crystalline forms of Paliperidone combined. For instance, the pure Form V can have less than 20% preferably less than 10%, more preferably less than 5%, and most preferably less than 1%, of Paliperidone Forms I and II combined.

The present invention further provides a process for the preparation of crystalline Form V of paliperidone comprising crystallization from a solution of paliperidone and a solvent selected from the group consisting of a mixture of acetone/water (3:1, volume ratio), n-propanol, and dioxane, and drying the obtained material under reduced pressure. Drying under reduced pressure preferably is at about 50°C to about 55°C for a sufficient period to obtain crystalline paliperidone form V, preferably overnight.

In the process for preparing Form V, the ingredients may be heated in order to achieve dissolution. Stirring may also be employed to promote dissolution. Preferably, the ingredients are heated to reflux. Where the ingredients are heated, the process may further comprise cooling, to induce crystallization. The obtained solid material may be washed, preferably with acetone. Further, these crystallization steps of dissolving paliperidone by heating in a solvent and cooling the heated solution followed by a washing step may be repeated prior to recovering the above crystalline paliperidone, preferably these crystallization steps are repeated three times. The crystalline form of paliperidone is recovered from the mixture by filtration. Where the solvent is n-propanol or dioxane and the mixture of paliperidone and solvent is heated to obtain a solution, the hot solution may be added dropwise into cold water, wherein the cold water preferably has a temperature of about 0°C to about 5°C.

The paliperidone crystalline Form V may be recovered by any method known to the skilled artisan. Preferably, the paliperidone crystalline Form V is recovered from the mixture by filtration, and then dried under reduced pressure (< 1 atmosphere).

The present invention also provides a process for preparing crystalline Form V of paliperidone, comprising drying crystalline Form IV of paliperidone to obtain Form V. Preferably, the drying is conducted under reduced pressure. More preferably, the drying is conducted under reduced pressure in an vacuum oven at about 50°C to about 60°C, e.g., at about 55°C, preferably overnight, followed by cooling to yield the crystalline Form V. Preferably, the cooling is to about room temperature.

The present invention also provides a process for preparing a mixture of crystalline Forms II and V of paliperidone, comprising heating crystalline Form III of paliperidone to about 110°C to about 130°C, preferably at about 120°C, followed by cooling to obtain the mixture. Preferably, the heating is conducted for about 15 minutes to about 1 hour, more preferably about 30 minutes. The heating can be conducted in ambient conditions. The cooling, preferably, is to room temperature.

The present invention also provides a process for preparing a mixture of crystalline Forms II and V of Paliperidone, comprising drying crystalline Form VI (to be described below) of Paliperidone to obtain the mixture. Preferably, the drying is conducted under reduced pressure. More preferably, the drying is conducted under reduced pressure in an vacuum oven at about 50°C to about 60°C, e.g., at about 55°C, preferably overnight, followed by cooling to yield the mixture of crystalline Forms II and V. Preferably, the cooling is to about room temperature.

The present invention also provides a process for preparing a mixture of crystalline Forms II and V of Paliperidone, comprising recrystallization of Paliperidone from acetone/water (volume ratio of about 1:5); and drying the solid product to obtain the mixture of Forms II and V.

The present invention also provides a process for preparing a mixture of solid Paliperidone comprising crystalline Form II, comprising recrystallization of Paliperidone from acetone/water (volume ratio about 1:1).

The present invention also provides a process for preparing a mixture of solid Paliperidone comprising crystalline Form II and amorphous Paliperidone solid, comprising recrystallization of Paliperidone from dichlorobenzene or propylene glycol.

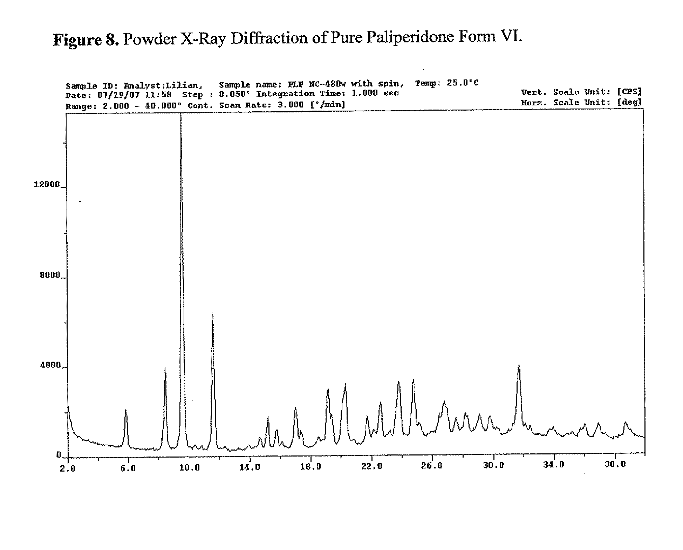

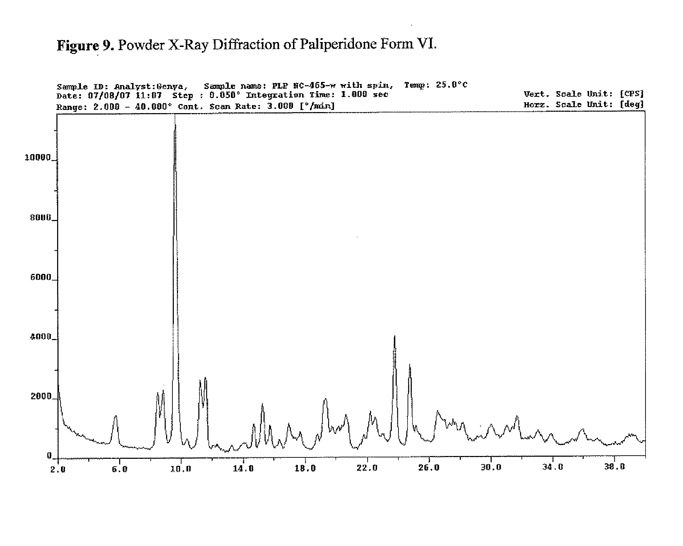

In another embodiment, the present invention provides a crystalline form of 9-hydroxy-risperidone (paliperidone), characterized by X-ray powder diffraction reflections at about: 5.8, 8.4, 9.5 and 11.6 degrees two theta ± 0.2 degrees two theta, designated Form VI. This crystalline form can be further characterized by one or more X-ray powder diffraction peaks at about 15.2, 24.8 and 31.7 degrees two-theta ± 0.2 degrees two-theta. Examples of powder x-ray diffraction patterns for the crystalline Form VI are shown in Figure 8 and 9.

The present invention further provides a process for the preparation of crystalline Form VI of paliperidone comprising crystallization from a solution of paliperidone and an ethanol/water mixture in a volume ratio of about 2:1 to about 4:1, preferably 3:1.

The present invention also provides a process for preparing a mixture of crystalline Form VI of Paliperidone, comprising recrystallization of Paliperidone from methanol/water (volume ratio of about 3:1) to yield Form VI.

In the processes for preparing Form VI, the ingredients may be heated in order to achieve dissolution. Stirring may also be employed to promote dissolution. Preferably, the ingredients are heated to reflux. Where the ingredients are heated, the process may further comprise cooling, to induce crystallization.

The powder X-ray diffraction patterns disclosed in this patent application were collected using an X-ray diffractometer with Cu radiation at λ = 1.5418 Å.

In yet another embodiment, the present invention provides pharmaceutical compositions comprising at least one of the above-described crystalline or amorphous forms of paliperidone and a pharmaceutically acceptable excipient.

Pharmaceutical compositions may be prepared as medicaments to be administered orally, parenterally, rectally, transdermally, buccally, or nasally. Suitable forms for oral administration include tablets, compressed or coated pills, dragees, sachets, hard or gelatin capsules, sub-lingual tablets, syrups, and suspensions. Suitable forms of parenteral administration include an aqueous or nonaqueous solution or emulsion, while for rectal administration, suitable forms for administration include suppositories with hydrophilic or hydrophobic vehicle. For topical administration, the invention provides suitable transdermal delivery systems known in the art, and for nasal delivery, there are provided suitable aerosol delivery systems known in the art.

In addition to the active ingredient(s), the pharmaceutical compositions of the present invention may contain one or more excipients or adjuvants. Selection of excipients and the amounts to use may be readily determined by the formulation scientist based upon experience and consideration of standard procedures and reference works in the field.

Diluents increase the bulk of a solid pharmaceutical composition, and may make a pharmaceutical dosage form containing the composition easier for the patient and care giver to handle. Diluents for solid compositions include, for example, microcrystalline cellulose (e.g. Avicel^{®}), microfine cellulose, lactose, starch, pregelitinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit^{®}), potassium chloride, powdered cellulose, sodium chloride, sorbitol, and talc.

Solid pharmaceutical compositions that are compacted into a dosage form, such as a tablet, may include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel^{®}), hydroxypropyl methyl cellulose (e.g. Methocel^{®}), liquid glucose, magnesium aluminum silicate, maltodextrin, methylcellulose, polymethacrylates, povidone (e.g. Kollidon^{®}, Plasdone^{®}), pregelatinized starch, sodium alginate, and starch.

The dissolution rate of a compacted solid pharmaceutical composition in the patient's stomach may be increased by the addition of a disintegrant to the composition. Disintegrants include alginic acid, carboxymethylcellulose calcium, carboxymethylcellulose sodium (e.g. Ac-Di-Sol^{®}, Primellose^{®}), colloidal silicon dioxide, croscarmellose sodium, crospovidone (e.g. Kollidon^{®}, Polyplasdone^{®}), guar gum, magnesium aluminum silicate, methyl cellulose, microcrystalline cellulose, polacrilin potassium, powdered cellulose, pregelatinized starch, sodium alginate, sodium starch glycolate (e.g. Explotab^{®}), and starch.

Glidants can be added to improve the flowability of a non-compacted solid composition and to improve the accuracy of dosing. Excipients that may function as glidants include colloidal silicon dioxide, magnesium trisilicate, powdered cellulose, starch, talc, and tribasic calcium phosphate.

When a dosage form such as a tablet is made by the compaction of a powdered composition, the composition is subjected to pressure from a punch and die. Some excipients and active ingredients have a tendency to adhere to the surfaces of the punch and die, which can cause the product to have pitting and other surface irregularities. A lubricant can be added to the composition to reduce adhesion and ease the release of the product from the die. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, and zinc stearate.

Flavoring agents and flavor enhancers make the dosage form more palatable to the patient. Common flavoring agents and flavor enhancers for pharmaceutical products that may be included in the composition of the present invention include maltol, vanillin, ethyl vanillin, menthol, citric acid, fumaric acid, ethyl maltol, and tartaric acid.

Solid and liquid compositions may also be dried using any pharmaceutically acceptable colorant to improve their appearance and/or facilitate patient identification of the product and unit dosage level.

In liquid pharmaceutical compositions of the present invention, the active ingredient and any other solid excipients are suspended in a liquid carrier such as water, vegetable oil, alcohol, polyethylene glycol, propylene glycol or glycerin.

Liquid pharmaceutical compositions may contain emulsifying agents to disperse uniformly throughout the composition an active ingredient or other excipient that is not soluble in the liquid carrier. Emulsifying agents that may be useful in liquid compositions of the present invention include, for example, gelatin, egg yolk, casein, cholesterol, acacia, tragacanth, chondrus, pectin, methyl cellulose, carbomer, cetostearyl alcohol, and cetyl alcohol.

Liquid pharmaceutical compositions of the present invention may also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth, and xanthan gum.

Sweetening agents such as sorbitol, saccharin, sodium saccharin, sucrose, aspartame, fructose, mannitol, and invert sugar may be added to improve the taste.

Preservatives and chelating agents such as alcohol, sodium benzoate, butylated hydroxy toluene, butylated hydroxyanisole, and ethylenediamine tetraacetic acid may be added at levels safe for ingestion to improve storage stability.

According to the present invention, a liquid composition may also contain a buffer such as gluconic acid, lactic acid, citric acid or acetic acid, sodium gluconate, sodium lactate, sodium citrate, or sodium acetate.

Selection of excipients and the amounts used may be readily determined by the formulation scientist based upon experience and consideration of standard procedures and reference works in the field.

The solid compositions of the present invention include powders, granulates, aggregates, and compacted compositions. The dosages include dosages suitable for oral, buccal, rectal, parenteral (including subcutaneous, intramuscular, and intravenous), inhalant, and ophthalmic administration. Although the most suitable administration in any given case will depend on the nature and severity of the condition being treated, the most preferred route of the present invention is oral. The dosages may be conveniently presented in unit dosage form and prepared by any of the methods well known in the pharmaceutical arts.

Dosage forms include solid dosage forms like tablets, powders, capsules, suppositories, sachets, troches, and losenges, as well as liquid syrups, suspensions, and elixirs.

The dosage form of the present invention may be a capsule containing the composition, preferably a powdered or granulated solid composition of the invention, within either a hard or soft shell. The shell may be made from gelatin, and, optionally, contain a plasticizer such as glycerin and sorbitol, and an opacifying agent or colorant.

The active ingredient and excipients may be formulated into compositions and dosage forms according to methods known in the art.

A composition for tableting or capsule filling can be prepared by wet granulation. In wet granulation, some or all of the active ingredients and excipients in powder form are blended, and then further mixed in the presence of a liquid, typically water, that causes the powders to clump into granules. The granulate is screened and/or milled, dried, and then screened and/or milled to the desired particle size. The granulate may then be tableted or other excipients may be added prior to tableting, such as a glidant and/or a lubricant.

A tableting composition can be prepared conventionally by dry blending. For example, the blended composition of the actives and excipients may be compacted into a slug or a sheet, and then comminuted into compacted granules. The compacted granules may subsequently be compressed into a tablet.

As an alternative to dry granulation, a blended composition may be compressed directly into a compacted dosage form using direct compression techniques. Direct compression produces a more uniform tablet without granules. Excipients that are particularly well suited for direct compression tableting include microcrystalline cellulose, spray dried lactose, dicalcium phosphate dihydrate and colloidal silica. The proper use of these and other excipients in direct compression tableting is known to those in the art with experience and skill in particular formulation challenges of direct compression tableting.

A capsule filling of the present invention may comprise any of the aforementioned blends and granulates that were described with reference to tableting, however, they are not subjected to a final tableting step.

In another embodiment, the present invention provides a method of treating a patient comprising administering to a patient in need thereof a therapeutically effective amount of the above crystalline form of paliperidone. Preferably, the patient suffers from a condition which may be treated with a norepinephrine or a serotonin re-uptake inhibitor. Such patient may be suffering from depression.

While it is apparent that the invention disclosed herein is well calculated to fulfill the objects stated above, it will be appreciated that numerous modifications and embodiments may be devised by those skilled in the art. Therefore, it is intended that the appended claims cover all such modifications and embodiments as falling within the true spirit and scope of the present invention. In the tables presented below in the Examples, "Wet" or "w" indicates analyzed after isolation, and "dry" or "d" means dried in a vacuum oven at 55°C overnight

### EXAMPLES

### Preparation of Paliperidone Form I

### Example 1:

A mixture of 3-(2-chloroethyl)-6,7,8,9-tetrahydro-9-hydroxy-2-methyl-4H-pyrrido[1,2-a]- pyrimidin-4-one (CMHTP, 4.393 g, 0.0168 mol), 6-fluoro-3-piperidino-1,2-benisoxazol (FPBI, 4.695 g, 0.0203 mol), sodium carbonate (4.968 g, 0.0422 mol) and potassium iodide (0.288 g, 0.0017 mol) in N,N-dimethylformamide (DMF, 50 ml) was heated for 8 h at 85°C. The mixture was poured into water (500 ml) and extracted with dichloromethane (4 x 100 ml). The extracts were combined, washed with water (4 x 100 ml), dried with anhydrous magnesium sulfate, filtered and evaporated under reduced pressure to afford the crude product. Crystallization from acetonitrile (100 ml) afforded 4.63 g of the product, 3-{2-[4-(6-fluoro-1,2-benzisoxazol-3-yl)-1-piperidinyl]ethyl}-9-hydroxy-2-methyl-4H-pyrrido[1,2-a]pyrimidin-4-one, in a chemical purity of more than 90%; Yield 58%. PXRD depicted 90% Form I and 10% Form V.

### Preparation of Paliperidone Form II

### Example 2:

A slurry of Paliperidone (2 g) in ethanol (52 ml.) was heated to reflux and the insoluble solid filtrated out. After filtration, the solution was cooled to room temperature and stirred for 5 hours. The mixture was cooled to 0-4 °C (ice bath) and stirred for an additional half hour. A solid was obtained by filtration and dried in a vacuum oven at 55°C for overnight to give 1.75 g of Paliperidone, characterized by X-ray powder diffraction reflections at about: 10.3, 14.6, 22.0, 24.6 and 25.0 degrees two theta ± 0.2 degrees two theta.

### Example 3:

A slurry of Paliperidone (2 g) in acetonitrile (36 ml.) was heated to reflux and the insoluble solid filtrated out. After filtration, the solution was cooled to room temperature and stirred for 5 hours. The mixture was cooled to 0-4 °C (ice bath) and filtered, washed with 40 ml. of acetonitrile and dried in a vacuum oven at 55°C for overnight to give 1.86 g of Paliperidone, characterized by X-ray powder diffraction reflections at about: 10.3, 14.6, 22.0, 24.6 and 25.0 degrees two theta ± 0.2 degrees two theta.

### Example 4:

A slurry of Paliperidone (2 g) in isopropanol (80 ml.) was heated to reflux and the insoluble solid filtrated out. After filtration, the solution was cooled to room temperature. The mixture was cooled to 0-4 °C (ice bath) and stirred for an additional hour. A solid was obtained by filtration and dried in a vacuum oven at 55°C for overnight to give 1.75 g of Paliperidone, characterized by X-ray powder diffraction reflections at about: 10.3, 14.6, 22.0, 24.6 and 25.0 degrees two theta ± 0.2 degrees two theta.

### Example 5:

A slurry of Paliperidone (1 g) in methanol (20 ml.) was heated to reflux and 0.05 g of activated carbon (SX-1) were added and stirred for 20 minutes at the same temperature. The mixture was filtered and the solution was cooled to room temperature and stirred for 2 hours. The mixture was cooled to 0-4 °C (ice bath) and stirred for an additional half hour. A solid was obtained by filtration and dried in a vacuum oven at 55°C for overnight to give 0.73 g of Paliperidone, characterized by X-ray powder diffraction reflections at about: 10.3, 14.6, 22.0, 24.6 and 25.0 degrees two theta ± 0.2 degrees two theta.

### Preparation of Paliperidone Form III

### Example 6:

A slurry of Paliperidone (1 g) in 1-methyl-2-pyrrolidone (5 ml.) was heated to 140 °C and the resulting solution was cooled to room temperature. The solid was filtrated and dried in a vacuum oven at 55 °C for overnight to give 0.53 g of Paliperidone, characterized by X-ray powder diffraction reflections at about: 10.8, 14.1, 15.8 and 16.8 degrees two theta ± 0.2 degrees two theta.

### Preparation of Paliperidone Form IV

### Example 7:

A slurry of Paliperidone (1 g) in 7 mL dioxane was heated to reflux temperature. Into the resulting solution was added at once 15 ml of water that were previously cooled in an ice bath. The solid was filtrated and analyzed by XRD to give Form IV.

### Example 8:

A slurry of Paliperidone (5 g) in a mixture of acetone/water 3:1 (200 ml) was heated to reflux and the resulting solution was cooled to 0°C. The slurry was vacuum filtrated and the resulting solid was crystallized again from 175 ml of the solvent mixture, and a third time from 100 ml. The solid was filtrated and analyzed by XRD to give Form IV.

### Example 9:

200mg of Paliperidone Form III was placed for 7 days in 100% relative humidity cell. The solid material was analyzed by XRD to give Form IV.

### Preparation of Paliperidone form V

### Example 10:

### a. Preparation of Paliperidone crude

A 250ml reactor equipped with a mechanical stirrer, a reflux condenser was charged under nitrogen with CMHTP, i.e., 3-(2-chloroethyl)-6,7,8,9-tetrahydro-9-hydroxy-2-methyl-4H-pyrrido[1,2-a]-pyrimidin-4-one, (20 gr), F-BIP, i.e., : 6-fluoro-3-piperidino-1,2-benisoxazole, (19.2 gr), Sodium carbonate (16 gr) and acetonitrile (200 ml). The suspension was heated to 65°C and stirred for 26.5 hours. The reaction mixture was cooled to -10°C, filtered under reduced pressure, and washed 3 times with acetonitrile (3 x 40ml each). The resulting solid was slurried in 200 ml water at room temperature, filtered under reduced pressure, washed 3 times with water (3 x 80 ml each), and with 40 ml acetone. The crude was dried in a vacuum oven at 50°C under reduced pressure for overnight to give 29 gr of Paliperidone crude.

### b. Crystallization of Paliperidone

A slurry of Paliperidone crude obtained in example 8a (28 gr) in a 1120 ml of a mixture of acetone/water (3:1) was heated to reflux till complete dissolution. After one hour, the solution was cooled to 0-4°C, filtrated, and washed with 60 ml of acetone. The procedure was repeated three times and finally the material was dried in a vacuum oven at 50°C under reduced pressure for overnight to give 15.2 gr of Paliperidone Form V.

### Example 11:

Hot solution of Paliperidone (1 gr) in n-Propanol (30 ml) was dropped wise added into ice bath cooled water (50 ml). The solid was filtrated and dried overnight in a vacuum oven at 55°C to give 0.57 gr of Paliperidone Form V.

### Example 12:

Hot solution of Paliperidone (1 gr) in dioxane (10 ml) was dropped wise added into ice bath cooled water (50 ml). The solid was filtrated and dried overnight in a vacuum oven at 55°C to give 0.52 gr of Paliperidone Form V.

### Example 13:

0.83 gr of Paliperidone Form IV was dried overnight at 55°C in a vacuum oven under reduced pressure. The solid material was cooled to room temperature and analyzed by XRD to give 0.45 gr of Paliperidone Form V.

### Example 14:

A mixture of 3-(2-chloroethyl)-6,7,8,9-tetrahydro-9-hydroxy-2-methyl-4H-pyrrido[1,2-a]- pyrimidin-4-one (CMHTP, 4.393 g, 0.0168 mol), 6-fluoro-3-piperidino-1,2-benisoxazol (FPBI, 4.695 g, 0.0203 mol), sodium carbonate (4.968 g, 0.0422 mol) and potassium iodide (0.288 g, 0.0017 mol) in N,N-dimethylformamide (DMF, 50 ml) was heated for 8 h at 85°C. The mixture was poured into water (500 ml) and extracted with dichloromethane (4 x 100 ml). The extracts were combined, washed with water (4 x 100 ml), dried with anhydrous magnesium sulfate, filtered and evaporated under reduced pressure to afford the crude product. Crystallization from acetonitrile (100 ml) afforded 4.63 g of the product, 3-{2-[4-(6-fluoro-1,2-benzisoxazol-3-yl)-1-piperidinyl]ethyl}-9-hydroxy-2-methyl-4H-pyrrido[1,2-a]pyrimidin-4-one, in a chemical purity of more than 90%; Yield 58%. PXRD depicted 90% Form I and 10% Form V.

### Preparation of Amorphous Paliperidone

### Example 15:

A slurry of Paliperidone Form II was slurried in n-Decane (1 mL) at 60°C for 24 hours. The solid was filtered and analyzed by XRD, to obtain amorphous Paliperidone mixed with Form II. The level of amorphous Paliperidone was about 40% [see Figure 1]

### Example 16:

### 5 g Paliperidon was dissolved in 85 ml of Dichloromethane at 40°C.

The solution was sprayed (5.6 ml/min) to the chamber with ambient nitrogen (30m³/h, 120°C) at co-current flow. The Atomizing flow (660 l/h) of nitrogen gave the Droplets affect which lead to the high evaporation rate. The temperature of the outlet solids were fixed to 80° C. The obtained sample was analyzed by XRD and found to be amorphous.

### Example 17:

### 5 g Paliperidon was dissolved in 85 ml of Dichloromethane at 40°C.

The solution was sprayed (5.6 ml/min) to the chamber with ambient nitrogen (30m³/h, 70°C) at co-current flow. The Atomizing flow (660 l/h) of nitrogen gave the Droplets affect which lead to the high evaporation rate. The temperature of the outlet solids were fixed to 45° C. The obtained sample was analyzed by XRD and found to be amorphous.

### Example 18:

A slurry of Paliperidone (1 g) in dichloromethane (17 ml) was heated to reflux until complete dissolution. The solvent was evaporated at 35°C under reduced pressure in a rotary evaporator. The solid was analyzed by PXRD to give pure amorphous Paliperidone. [see Figure 2]

### Preparation of Pure Paliperidone Form I

### Example 19:

A 100 ml flask equipped with a mechanical stirrer, a reflux condenser was charged under nitrogen with CMHTP (2 g), F-BIP (1.92 g), sodium carbonate (1.6 g), potassium iodide (0.03 g) and isopropyl alcohol (20 ml). The suspension was heated to 65°C and stirred for 24 hours to obtain yellowish slurry. The reaction mixture was cooled to -10°C in 2 hours, then filtered under reduced pressure and rinsed with 3 portions of isopropyl alcohol (10 ml each). The resulting solid was reslurried 3 times with water (20 ml each time) and 3 times with acetone (10 ml each), filtered and dried at room temperature for 1 hour and at 60°C under reduced pressure for 1 hour to obtain Paliperidone (1.84 g, 57.7%). The solid was analyzed by XRD to be form 1.

### Preparation of Paliperidone Form VI

### Example 20:

A slurry of Paliperidone (1 gr) in a mixture of methanol/water 3:1 (37 ml) was heated to reflux temperature until complete dissolution was achieved. The resulting solution was cooled to 0°C. The solid was vacuum filtrated and analyzed by XRD to give Form VI.

### Preparation of Paliperidone Mixtures

**Example 21**: A mixture of 3-(2-chloroethyl)-5,7,8,9-tetrahydro-9-hydroxy-2-methyl-4H-pyrrido[1,2-a]-pyrimidin-4-one (CMHTP, 4.393 g, 0.0168 mol), 6-fluoro-3-piperidino-1,2-benisoxazol (FPBI, 4.695 g, 0.0203 mol), sodium carbonate (4.968 g, 0.0422 mol) and potassium iodide (0.288 g, 0.0017 mol) in N,N-dimethylformamide (DMF, 50 ml) was heated for 8 h at 85°C. The mixture was poured into water (500 ml) and extracted with dichloromethane (4 x 100 ml). The extracts were combined, washed with water (4 x 100 ml), dried with anhydrous magnesium sulfate, filtered and evaporated under reduced pressure to afford the crude product. Crystallization from acetonitrile (100 ml) afforded 4.63 g of the product, 3-{2-[4-(6-fluoro-1,2-benzisoxazol-3-yl)-1-piperidinyl]ethyl}-9-hydroxy-2-methyl-4H-pyrrido[1,2-a]pyrimidin-4-one, in a chemical purity of more than 90%; Yield 58%. PXRD depicted 90% Form I and 10% Form V.

### Example 22:

200mg of Paliperidone Form III was heated to 120°C for 30 min in ambient conditions. The solid material was cooled to RT and analyzed by XRD to give a mixture of Forms V and II.

### Example 23:

1.44 gr of Paliperidone Form VI was dried overnight at 55°C in a vacuum oven under reduced pressure. The solid material was cooled to RT and analyzed by XRD to give a mixture of 0.66gr Paliperidone Forms V and II.

### Example 24: Preparation of Paliperidon Form I by slurry in different solvents.

Slurry of Paliperidone in the indicated volumes of the indicated solvents was stirred at the indicated temperatures and the indicated times. The solid was collected by vacuum filtration, dried where is indicated (in a vacuum oven at 55°C for overnight) and analyzed. The results are displayed in the next table.

| **Starting form** | **solvent** | **Solvent volume (ml/g)** | **Stirring temp.** | **Stirring time** | **Wet / dry** |
|---|---|---|---|---|---|
| II | Ethanol / waster 1:1 | 10 | 60°C | 26h | w |
| II | dichlorobenzen | 10 | 60°C | 26h | w |
| II | Acetone / water 1:1 | 10 | 60°C | 26h | w |
| I | IPA | 10 | 60°C | 17h | w+d |
| I | water | 10 | 60°C | 24h | w+d |
| I | acetone | 10 | 25°C | 47h | w+d |
| I | IPA | 10 | 25°C | 47h | w+d |
| I | water | 10 | 25°C | 47h | w+d |

### Example 25: Preparation of Paliperidone Form I by addition of a different hot solvent.

Slurry of Paliperidone in 17 volumes of dichloromethane was heated to reflux until complete dissolution. The hot solution was added dropwise into 50 volumes of hexane that was previously cooled in an ice bath. The resulting solid was collected by vacuum filtration, dried in a vacuum oven at 55 °C, and analyzed to give Form I.

### Example 26: Preparation of Paliperidone Form I.

### a. Preparation of Paliperidone crude

A 250 ml reactor equipped with a mechanical stirrer, a reflux condenser was charged under nitrogen with CMHTP (50 g), F-BIP (48 g), Sodium carbonate (40 g) and isopropanol (500 ml). The suspension was heated to 65°C and stirred for 25. The reaction mixture was cooled to -5 °C, filtered under reduced pressure, and washed 2 times with 200 ml water, followed by 200 ml IPA. The crude was dried in a vacuum oven at 50°C under reduced pressure for overnight to give Paliperidone crude.

### b. Crystallization of Paliperidone in methanol

A slurry of 2 g of Paliperidone (obtained in section a) in 20 ml methanol was heated to reflux and an additional 20 ml of methanol were added (foreign bodies were removed from the mixture). After cooling to room temperature the mixture was stirred for overnight. The material was filtrated and dried in a vacuum oven at 50 oC under reduced pressure for overnight to give Paliperidone Form I.

### c. Crystallization of Paliperidone in acetonitrile

A slurry of 2 g of Paliperidone (obtained in section a) in 36 ml acetonitrile was heated to reflux. After cooling to room temperature the mixture was cooled to 0 °C. The material was filtrated, washed with acetonitrile and dried in a vacuum oven at 50 oC under reduced pressure for overnight to give Paliperidone Form I.

### Example 27: Preparation of Paliperidone Form II by crystallization.

A slurry of Paliperidone, in the indicated solvent, at the indicated volumes was heated to the indicated temperatures until complete dissolution, wherein each of the ratios presented in the table below represents volume ratio of the two solvents named immediately preceding the ratio. After the compound was dissolved, the oil bath was removed and the solution was cooled to room temperature (excepted were is indicated). The solid was filtrated, dried in a vacuum oven at 55°C for overnight, and analyzed as shown in the next table.

| **solvent** | **Solvent vol. (ml/g)** | **Heating temp.** | **Cooling temp.** | **Wet / dry** |
|---|---|---|---|---|
| ethanol | 28 | reflux | r.t. | w+d |
| n-propanol | 20 | reflux | r.t. | w+d |
| IPA | 42 | reflux | r.t. | w+d |
| methanol | 48 | reflux | r.t. | w+d |
| toluene | 15 | reflux | r.t. | w+d |
| Iso-butyl acetate | 55 | reflux | r.t. | w+d |
| n-butanol | 20 | reflux | r.t. | w+d |
| MIBK | 39 | reflux | r.t. | w+d |
| 2-pentanol | 22 | reflux | r.t. | w+d |
| PGME | 8 | reflux | r.t. | w+d |
| n-butyl acetate | 31 | reflux | r.t. | w+d |
| Diethyl carbonate | 28 | reflux | r.t. | w+d |
| chlorobenzen | 6 | reflux | r.t. | w+d |
| Cellosolve | 8 | reflux | r.t. | w+d |
| n-pentanol | 15 | reflux | r.t. | w+d |
| THF | 24 | reflux | r.t. | w+d |
| 1,2-dichloroethane | 12 | reflux | r.t. | w+d |
| DMF | 5 | reflux | r.t. | w+d |
| Dimethyl acetamide | 5 | reflux | r.t. | d |
| Dichlorobenzen | 5 | reflux | r.t. | w+d |
| Propylene glycole | 5 | reflux | r.t. | w+d |
| DMSO | 5 | reflux | r.t. | w+d |
| DMC | 33 | reflux | r.t. | w+d |
| 2-butanol | 20 | reflux | r.t. | w+d |
| MIPK | 54 | reflux | r.t. | w+d |
| Iso-butanol | 26 | reflux | r.t. | w+d |
| Ethanol/water 3:1 | 12 | reflux | r.t. | w+d |
| Ethanol/water 1:1 | 36 | reflux | r.t. | w+d |
| MEK | 69 | reflux | r.t. | w+d |
| acetonitrile | 100 | reflux | r.t. | w+d |
| EtOAc/water 3:1 | 50 | reflux | r.t. | w+d |
| EtOAc/water 1:1 1 | 55 | reflux | r.t. | w+d |
| dioxane | 6 | reflux | r.t. | w+d |
| Dibutyl ether | 165 | reflux | r.t. | w+d |
| acetone | 155 | reflux | r.t. | w+d |
| Acetone/water 3:1 | 25 | reflux | r.t. | w+d |
| Acetonitril/water 1:1 | 40 | reflux | r.t. | w+d |
| n-butanol | 23 | 135°C | r.t. | w+d |
| cellosolve | 8 | 115°C | r.t. | w+d |
| chlorobenzen | 7 | 115°C | r.t. | w+d |
| DMSO | 5 | 110°C | r.t. | w+d |
| Dibutyl ether | 140 | 130°C | r.t. | w+d |
| PGME | 7 | 130°C | r.t. | w+d |
| Iso-butyl acetate | 35 | reflux | r.t. | w+d |
| n-propanol | 30 | 90°C | r.t. | w+d |
| ethanol | 80 | 70°C | r.t. | w |
| IPA/water 1:1 | 19 | reflux | 0°C | w+d |
| IPA/water 3:1 | 10 | reflux | 0°C | w+d |

### Example 28: Preparation of Paliperidone Form II by addition of a different solvent

Slurry of Paliperidone in 20 volumes (ml/g) of dichloromethane was heated to reflux until complete dissolution. The solution was cooled to room temperature and the indicated anti-solvent was gradually added until precipitation. The mixture was stirred at room temperature for 1.5 h and the solid was collected by vacuum filtration (dried in a vacuum oven at 55 °C for overnight where is indicated) and analyzed as shown in the next table.

| **Anti-solvent** | **Anti-Solvent volume (ml/g)** | **Wet / dry** |
|---|---|---|
| MTBE | 15 | w+d |
| MEK | 20 | w+d |
| EtOAc | 20 | w+d |
| Acetonitril | 25 | w+d |
| Cyclohexane | 30 | w+d |
| heptane | 15 | w+d |
| toluene | 15 | w+d |

### Example 29: Preparation of Paliperidone Form II by addition of a different solvent at a different temperature.

Slurry of Paliperidone in the indicated volumes of the indicated solvent was heated to reflux until complete dissolution. The cooled anti-solvent was added at once. The resulting solid was collected by vacuum filtration (dried in a vacuum oven at 55 °C for overnight where is indicated), and analyzed as shown in the next table, wherein "Wet" indicates analyzed after isolation, and "dry" means dried in a vacuum oven at 55°C overnight.

| **solvent** | **Total solvent vol. (ml/g)** | **Anti-solvent** | **Anti-solvent vol. (ml/g)** | **Wet / dry** |
|---|---|---|---|---|
| toluene | 15 | hexane | 35 | w |
| butanol | 7 | water | 70 | d |

### Example 30: Preparation of Paliperidone Form III by slurry in NMP.

Slurry of Paliperidone Form II in 10 volumes of NMP the indicated volumes was stirred for 24 h at 60°C. The solid was collected by vacuum filtration, and analyzed to give Form III.

### Example 31: Preparation of Paliperidone Form IV by addition of a different solvent at a different temperature.

A slurry of Paliperidone (1g) in 7 ml n-butanol was heated to reflux temperature. Into the resulting solution was added at once 70 ml. of water that were previously cooled in an ice bath. The solid was filtrated and analyzed by XRD to give Form IV.

### Example 32: Preparation of Paliperidone Form IV by crystallization.

A slurry of Paliperidone, in the indicated solvent, at the indicated volumes was heated to the indicated temperatures until complete dissolution, wherein each of the ratios presented in the table below represents volume ratio of the two solvents named immediately preceding the ratio. After the compound was dissolved, the oil bath was removed and the solution was cooled to room temperature (excepted were is indicated). The solid was filtrated and analyzed as shown in the next table.

| **solvent** | **Solvent vol. (ml/g)** | **Heating temp.** | **Cooling temp.** |
|---|---|---|---|
| Acetone / water 5:1 | 40 | reflux | r.t. |
| Acetone / water 3:1 | 40 | reflux | 0°C |
| methanol / water 3:1 | 34 | reflux | 0°C |

### Example 33: Preparation of Paliperidone Form IV by addition of a different solvent at a different temperature.

Slurry of Paliperidone in the indicated solvent was heated to reflux until complete dissolution. The hot solution was added dropwise into an anti-solvent that was previously cooled in an ice bath. The resulting solid was collected by vacuum filtration, and analyzed as shown in the next table.

| solvent | Solvent vol. (ml/g) | Anti-solvent | Anti-solvent vol. (ml/g) |
|---|---|---|---|
| n-propanol | 30 | water | 50 |
| dioxane | 10 | water | 50 |

### Example 34: Preparation of Paliperidone Form IV by filtration through activated carbon

A slurry of Paliperidone in 40 volumes (i.e., g/40 ml) of acetone/water (3:1, volume ratio) was heated to reflux until complete dissolution. After the compound was dissolved, the hot solution was filtrated through hi-flow and cooled in an ice bath. The solid was filtrated and analyzed as shown in the next table.

| **Type of active carbon** | **Time of reflux** | **Time of cooling** |
|---|---|---|
| HB ultra | 40 min | 60 min |
| CGP super | 60 min | 50 min |
| GBG | 65 min | 50 min |
| SX plus | 55 min | 60 min |
| ROX 0.8 | 55 min | 2h |
| A super eur | 60 min | 2h |

### Example 35: Preparation of mixture of Paliperidone by crystallization.

A slurry of Paliperidone, in the indicated solvent, at the indicated volumes was heated to the indicated temperatures until complete dissolution, wherein each of the ratios presented in the table below represents volume ratio of the two solvents named immediately preceding the ratio. After the compound was dissolved, the oil bath was removed and the solution was cooled to room temperature (excepted were is indicated). The solid was filtrated and analyzed as shown in the next table.

| solvent | Solvent vol. (ml/g) | Heating temp. | Cooling temp. | Wet | dry |
|---|---|---|---|---|---|
| Acetone / water 1:1 | 98 | reflux | 0°C | II + 9.7, 10.9, 15.8, 21.2, 26.0 | II ??? |
| dichlorobenzen | 5 | 120°C | r.t. | Am. + II | |
| Propylene glycole | 7 | 120°C | r.t. | II + Am. | |
| Acetone / water 1:5 | 40 | reflux | 0°C | | II + V |
| methanol / water 3:1 | 34 | reflux | 0°C | VI | V+II |

## Claims

1. A crystalline form of Paliperidone, **characterized by** data selected from the group consisting of:
(i) X-ray powder diffraction spectrum with four or more peaks from the list of: about 9.8, 10.9, 15.8, 21.2 and 21.6 degrees two theta ± 0.2 degrees two theta;
(ii) a solid-state ¹³C NMR spectrum with signals at about 163.4, 161.4 and 157.9 ± 0.2 ppm; and
(iii) a solid-state ¹³C NMR spectrum having chemical shifts differences between the signal exhibiting the lowest chemical shift and another in the chemical shift range of 100 to 180 ppm of about 51.1, 49.1 and 45.6 ± 0.1 ppm.

2. The crystalline form of claim 1, wherein the X-ray powder diffraction spectrum further has one or more peaks from the following: about 14.1, 18.0 and 26.0 ± 0.2 degrees two theta.

3. The crystalline form of claim 1, wherein the solid state ¹³C NMR spectrum further has one or more signals at 119.5 and 112.3 ± 0.2 ppm.

4. The crystalline form of claim 3, wherein the solid state ¹³C NMR spectrum is substantially as depicted in Figures 6 and 7.

5. The crystalline form of claim 1, wherein the X-ray powder diffraction spectrum is substantially as depicted in Figures 15.

6. The crystalline form of claim 1, wherein the form is anhydrous.

7. The crystalline form of claim 6, wherein the water content is about 0.3% by weight, as measured by Karl Fischer titration.

8. The crystalline form of claim 1, having less than 20% by weight of any one of other crystalline forms of paliperidone.

9. The crystalline form of Paliperidone of claim 8, having less than 20% by weight of any one of crystalline forms of paliperidone having an X-ray powder diffraction spectrum with peaks at about: 10.1, 12.4, 14.3, 17.0 and 17.2 degrees two theta ± 0.2 degrees two theta; or an X-ray powder diffraction spectrum with peaks at about: 10.3, 14.6, 22.0, 24.6 and 25.0 degrees two theta ± 0.2 degrees two theta or an X-ray powder diffraction spectrum with peaks at about: 10.3, 13.3, 13.9, 14.6, and 15.1 degrees two theta ± 0.2 degrees two theta.

10. The crystalline form of Paliperidone of claim 9, having less than 10% by weight of any one of crystalline forms of paliperidone having an X-ray powder diffraction spectrum with peaks at about: 10.1, 12.4, 14.3, 17.0 and 17.2 degrees two theta ± 0.2 degrees two theta; or an X-ray powder diffraction spectrum with peaks at about: 10.3, 14.6, 22.0, 24.6 and 25.0 degrees two theta ± 0.2 degrees two theta, or an X-ray powder diffraction spectrum with peaks at about: 10.3, 13.3, 13.9, 14.6, and 15.1 degrees two theta ± 0.2 degrees two theta.

11. The crystalline form of Paliperidone of claim 10, having less than 5% by weight of any one of crystalline forms of paliperidone having an X-ray powder diffraction spectrum with peaks at about: 10.1, 12.4, 14.3, 17.0 and 17.2 degrees two theta ± 0.2 degrees two theta; or an X-ray powder diffraction spectrum with peaks at about: 10.3, 14.6, 22.0, 24.6 and 25.0 degrees two theta ± 0.2 degrees two theta or an X-ray powder diffraction spectrum with peaks at about: 10.3, 13.3, 13.9, 14.6, and 15.1 degrees two theta ± 0.2 degrees two theta.

12. The crystalline form of Paliperidone of claim 11, having less than 1% by weight of any one of crystalline forms of paliperidone having an X-ray powder diffraction spectrum with peaks at about: 10.1, 12.4, 14.3, 17.0 and 17.2 degrees two theta ± 0.2 degrees two theta; or an X-ray powder diffraction spectrum with peaks at about: 10.3, 14.6, 22.0, 24.6 and 25.0 degrees two theta ± 0.2 degrees two theta an X-ray powder diffraction spectrum with peaks at about: 10.3, 13.3, 13.9, 14.6, and 15.1 degrees two theta ± 0.2 degrees two theta.

13. A process for preparing crystalline paliperidone as defined in any one of the preceding claims comprising:
(a) crystallization from a solution of paliperidone and a mixture of acetone/water (3:1, volume ratio), and drying the obtained material under reduced pressure; or
(b) drying a crystalline form of paliperidone **characterized by** X-ray powder diffraction reflections at about: 10.2, 12.2 and 15.5 degrees two theta ± 0.2 degrees two theta to obtain crystalline paliperidone as defined in any one of the preceding claims.
